# EUROPEAN PATENT SPECIFICATION

(11) **EP 0 832 573 B1**
(45) Date of publication and mention of the grant of the patent: **12.06.2002**
(21) Application number: 97307716.7
(22) Date of filing: 30.09.1997
(51) Int. Cl.: A44B 18/00, A61F 13/15

(54) **Surface fastener and paper diaper using the surface fastener**
Flächenhaftverschluss und Windel mit diesem Haftverschluss
Fermeture à surface d'accrochage et couche comportant une telle fermeture

(30) Priority: 30.09.1996 JP 25925796
(43) Date of publication of application: 01.04.1998
(73) Proprietor: YKK CORPORATION, Chiyoda-ku, Tokyo (JP)
(72) Inventor: Minato, Hitomi, Toyama-shi, Toyama-ken (JP)
(74) Representative: Luckhurst, Anthony Henry William

(56) References cited:
- EP-A- 0 324 578
- EP-A- 0 693 889
- WO-A-95/03723
- GB-A- 1 181 022
- US-A- 4 322 875

## Description

### BACKGROUND OF THE INVENTION

### 1. Description of the Invention:

This invention relates to a surface fastener to be used as a fastener of a disposable paper diaper, the surface fastener being improved in its attaching means so that the paper diaper is easy to handle.

### 2. Description of the Related Art:

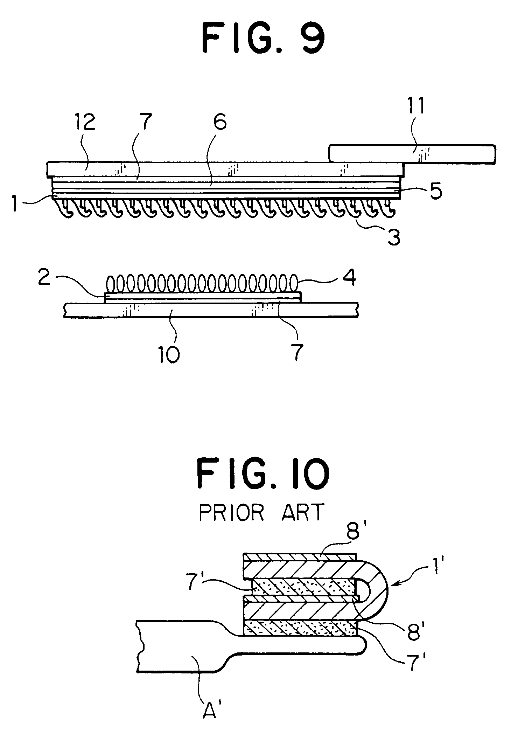

FIG. 10 of the accompanying drawings shows a paper diaper fastener which is disclosed in Japanese Utility Model Publication No. Hei 1-29230. In this known fastener, a plastic substrate tape 1' has on its one surface an adhesive layer 7' extending from the center to one end and a protective peel portion 8' extending from the center to the other end. On the other surface, the plastic substrate tape 1' has another protective peel portion 8' extending from the center to one end and another adhesive layer 7' extending from the center to the other end, which is a reverse arrangement to that on the one surface. The plastic substrate tape 1' is attached in a folded form to a paper diaper A' and, in use, the adhesive layer 7' is peeled off the protective peel portion 8' and is then adhered to a companion attaching section of the diaper A'.

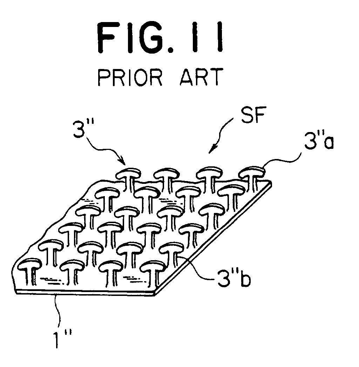

Japanese Patent Laid-Open Publication No. Hei 2-5947 discloses a concept of using a surface fastener SF in a disposable diaper. As shown in FIG. 11, in the surface fastener, a thin strong flexible substrate plate has on its front surface a multiplicity of resilient mushroom-shaped engaging elements 3" each having a stem 3"b projecting upright from the front surface and a round head 3"a at the top of the stem 3"b. This surface fastener is fixed to an end of a flexible elongated rectangular polymer tab and is provided with a low-viscosity pressure-sensitive adhesive agent so that it is kept in a folded form; in use, the surface fastener is unfolded and is then fastened with a companion surface fastener.

According to the first-named conventional diaper fastener in a form of the plastic tape 1', since the diaper A' is fastened by the adhesive layer 7', it is impossible to fasten the diaper A' with adequate firmness. Further, the adhesive layer 7' of the plastic tape 1' tends to touch the infant's skin which is very delicate when the diaper A' is in use, so that the infant might suffer atopic dermatitis and other skin disease.

According to the second-named conventional diaper fastener in a form of a surface fastener, since the engaging elements 3" of the surface fastener SF have a very special shape, it is inevitable to use a very complicated processing means in manufacturing the surface fastener SF, and there is a danger that the pressure-sensitive adhesive agent on the polymer tab might tough the infant's skin to cause atopic dermatitis or other skin disease when the diaper is in use.

US-A- 4 322 875 discloses a surface fastener in which the fastener parts are adhered to respective objects to be joined by a common pressure sensitive adhesive.

### SUMMARY OF THE INVENTION

A first object of this invention is to provide a surface fastener which is suitable for a disposable paper diaper and in which either an adhesive layer or an adhesive double coated tape has such a form as not to contact an infant' s skin directly and which is easy to handle when the used paper diaper is to be cast off, at which time the used diaper is folded into a compact size and is wrapped in a simple manner.

A second object of the invention is to provide a surface fastener which is very simple to attach and handle with respect to a paper diaper.

A third object of the invention is to provide a surface fastener which is shaped into a form suitable for use in a paper diaper in view of relation between the adhesive strength of an adhesive layer and an adhesive double coated tape with respect to the surface fastener and the shape of engaging elements.

A fourth object of the invention is to provide a surface fastener which has a shape suitable for use in a paper diaper by specifying the surface fastener manufacturing means and shape.

A fifth object of the invention is to provide a surface fastener of which hook-shaped engaging elements are suitable for a paper diaper by specifying the hook-shaped engaging element manufacturing means.

A sixth object of the invention is to provide a paper diaper to which a surface fastener is attached in an optimal form and in which the surface fastener has such a shape that an adhesive layer and an adhesive double coated tape will not come into direct contact with an infant's skin.

A seventh object of the invention is to provide a paper diaper in which a surface fastener can be attached in a simple manner and which can be handled simply when it is to be cast off after using.

According to a first aspect of the invention, there is provided a surface fastener comprising: a first substrate sheet having on its front surface a multiplicity of first engaging elements and on its rear surface a first adhesive layer; a second substrate sheet having on its front surface a multiplicity of second engaging elements engageable with the first engaging elements and on its rear surface a second adhesive layer; the first adhesive layer having an adhesive strength smaller than that of said second adhesive layer.

Preferably, the first adhesive layer is an adhesive double coated tape and has on its outer surface a backing sheet adhered thereto, while the second adhesive layer has on its outer surface a peel paper adhered thereto.

Preferably the backing sheet has on its outer surface another adhesive layer to an outer surface of which another peel paper is adhered.

Preferably, the second engaging elements of the second substrate sheet on which the first-mentioned adhesive layer is disposed are loop-shaped engaging elements, and the first engaging elements of the first substrate sheet on which the adhesive double coated tape is disposed are hook-shaped engaging elements.

Preferably, at least one of the first and second substrate sheets is knitted or woven, and the engaging elements of one of the first and second substrate sheets are hook-shaped engaging elements and/or loop-shaped engaging elements.

Preferably, hook-shaped engaging elements are molded on the front surface of the first substrate sheet by injection molding.

According to another aspect of the invention, there is provided a paper diaper having a front covering portion, a hip covering portion having attachment strips on opposite sides thereof, and a plurality of surface fasteners, each surface fastener comprising: a first substrate sheet having on its front surface a multiplicity of first engaging elements; a second substrate sheet having on its front surface a multiplicity of second engaging elements engageable with the first engaging elements and on its rear surface an adhesive layer which adheres said substrate sheet to said front covering portion; and an adhesive double coated tape adhered to a rear surface of the first substrate sheet with an adhesive strength which is smaller than that of the adhesive layer, the adhesive double coated tape being adhered to a respective attachment strip.

Preferably, a backing sheet is adhered to an outer surface of the adhesive double coated tape and has on a surface opposite to the tape an additional adhesive layer of which outer surface is adhered to the respective attachment strip of the hip covering portion.

Preferably, the second engaging elements of each second substrate sheet on which the first-mentioned adhesive layer is disposed are loop-shaped engaging elements, and the first engaging elements of each first substrate sheet on which the adhesive double coated tape is disposed are hook-shaped engaging elements.

Preferably, the first or second substrate sheet is knitted or woven, and the engaging elements of the first or second substrate sheets are hook-shaped engaging elements and/or loop-shaped engaging elements.

Preferably, the hook-shaped engaging elements are molded on the front surface of the first substrate sheet by injection molding.

### BRIEF DESCRIPTION OF THE DRAWINGS

FIG. 1 is a front view of a surface fastener according to a first embodiment of this invention;
FIG. 2 is a front view of a modified surface fastener according to a second embodiment of the invention;
FIG. 3 is a perspective view showing a paper diaper in an opened posture;
FIG. 4 is a perspective view showing the paper diaper in a fastened posture;
FIG. 5 is a side view showing one example of use of the surface fastener of FIG. 1;
FIG. 6 is a side view showing one example of use of the surface fastener of FIG. 2;
FIG. 7 is a side view showing the manner in which the surface fastener of FIG. 6 is separated;
FIG. 8 is a side view showing one example of use of another modified surface fastener according to a third embodiment of the invention;
FIG. 9 is a side view showing one example of use of an injection-molded surface fastener according to a fourth embodiment;
FIG. 10 is a fragmentary cross-sectional view of a conventional surface fastener used in a paper diaper; and
FIG. 11 is a fragmentary perspective view of another conventional surface fastener used in a paper diaper.

### DETAILED DESCRIPTION OF THE PREFERRED EMBODIMENTS

Surface fasteners according to preferred embodiments and paper diapers using the surface fasteners will now be described in detail with reference to the accompanying drawings.

Recently application of surface fasteners, instead of adhesive tapes, to paper diapers has been on the increase. One of the reasons is that many infants having delicate skins suffer atopic dermatitis when they touch the adhesive tapes.

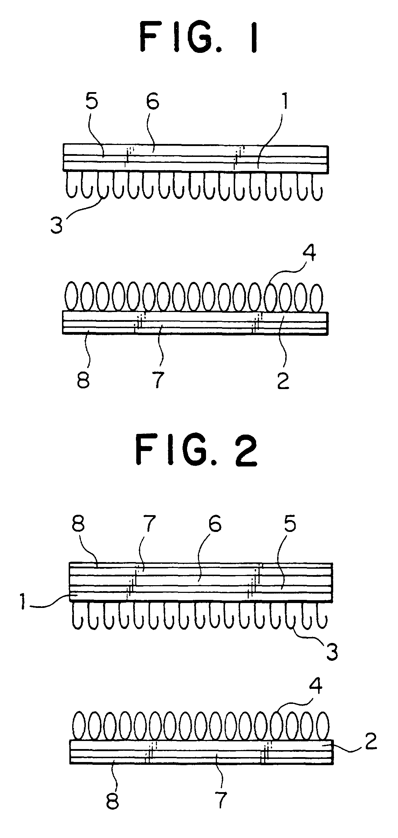

FIG. 1 shows a surface fastener according to a first embodiment of this invention which is designed in such a manner that an adhesive layer and an adhesive double coated tape would not tough the infant's skin when the surface fastener is used for a paper diaper. In the surface fastener, a male surface fastener member includes a first substrate sheet 1 having on its front surface a multiplicity of hook-shaped engaging elements 3, an adhesive double coated tape 5 adhered to a rear surface of the first substrate sheet 1, and a backing sheet 6 adhered to an outer surface of the tape 5. A female surface fastener member includes a second substrate sheet 2 having on its front surface a multiplicity of loop-shaped engaging elements 4, an adhesive layer 7 on a rear surface of the second substrate sheet 2, and a peel paper 8 adhered to an outer surface of the adhesive layer 7.

FIG. 2 shows an alternative surface fastener according to a second embodiment of the invention. In the modified surface fastener, a male surface fastener member includes a first substrate sheet 1 having on its front surface a multiplicity of hook-shaped engaging elements 3, an adhesive double coated tape 5 on a rear surface of the substrate sheet 1, a backing sheet 6 adhered to an outer surface of the adhesive double coated tape 5, an adhesive layer 7 on an outer surface of the backing sheet 6, and a peel taper 8 adhered to an outer surface of the adhesive layer 7. In the meantime, a female surface fastener member includes a second substrate sheet 2 having on its front surface a multiplicity of loop-shaped engaging elements 4, an adhesive layer 7 on a rear surface of the substrate sheet 2, a peel paper 8 adhered to an outer surface of the adhesive layer 7.

In the foregoing surface fasteners, the first and second substrate sheets 1, 2 are woven or knitted of synthetic fibers such as polyamide or polyester and, at the same time, the hook-shaped engaging elements 3 are formed from monofilament while the loop-shaped engaging elements 4 are formed from multifilament. The backing sheet 6 is made of thermoplastic resin. The adhesive double coated tape 5 and the adhesive layer 7 are made of rubber resin, acrylic resin, etc. The adhesive double coated tape 5 has an adhesive strength smaller than that of the adhesive layer 7.

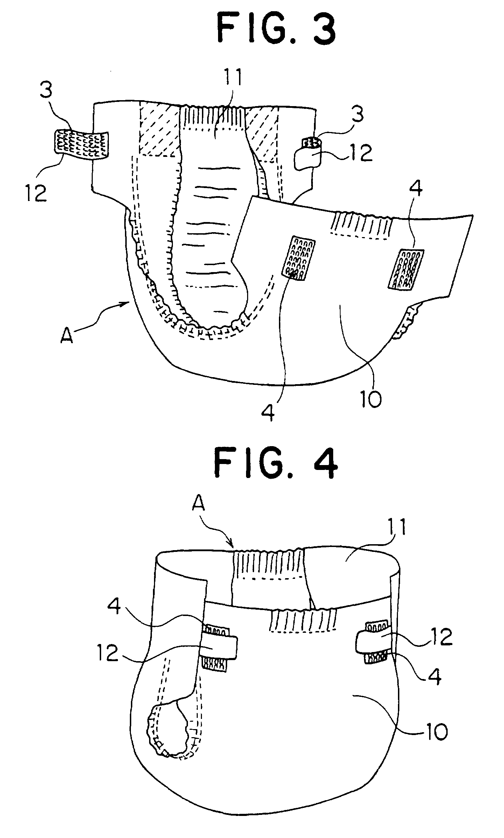

FIGS. 3 and 4 respectively shows an example in which the foregoing surface fasteners are used in a paper diaper A. A pair of female surface fastener members are attached to the outer surface of a front covering portion 10 of the paper diaper A at its opposite sides, while a pair of male surface fastener members are attached to inner surfaces of a pair of attachment strips 12 fixed to opposite sides of a hip covering portion 11 of the paper diaper A.

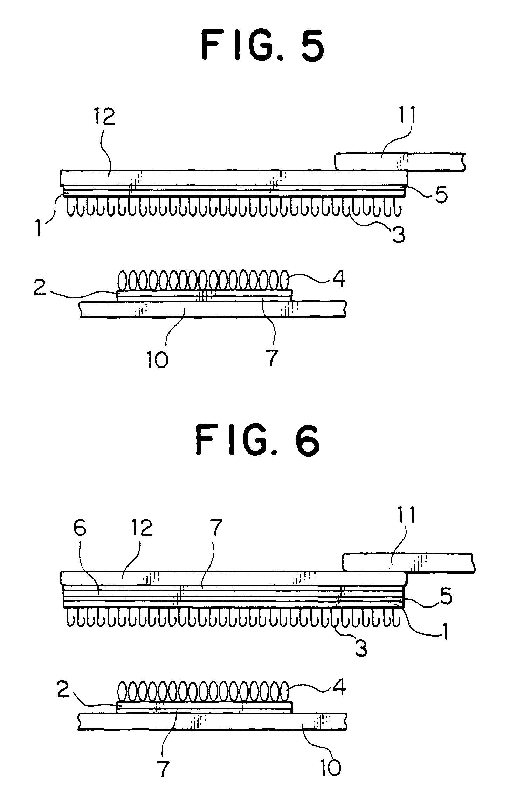

FIG. 5 shows one example in which the surface fastener of FIG. 1 is used in a paper diaper A. In this example, the second substrate sheet 2 of each female surface fastener is attached to each of opposite sides of the front covering portion 10 of the paper diaper A as shown in FIG. 5 by the adhesive layer 7 with the peel paper 8 peeled off the rear surface thereof. In the meantime, the first substrate sheet 1 of each male surface fastener of FIG. 1 is adhered to each of the attachment strips 12 of the hip covering portion 11 of the paper diaper A by the adhesive double coated tape 5 with the backing sheet 6 peeled off the rear surface thereof. In using the diaper A, the hook-shaped engaging elements 3 of the first substrate sheet 1 are brought into engagement with the loop-shaped engaging elements 4 of the second substrate sheet 2 as the female surface fastener is pressed against the corresponding male surface fastener. In an alternative form, a pair of backing sheets 6 may be attached directly to the hip covering portion 11 for substitution for the attachment strips 12.

For casting off the paper diaper A after using, with the hook-shaped engaging elements 3 kept in engagement with the loop-shaped engaging elements 4, each attachment strip 12 is pulled outwardly so as that the adhesive double coated tape 5 is peeled off the first substrate sheet 1 and hence stay on the attachment strip 12, and then the paper diaper A is folded, whereupon the paper diaper A is wrapped in a compact size by adhering the adhesive double coated tapes 5 of the attachment strips 12 to the outer surface of the folded paper diaper A at some positions thereof.

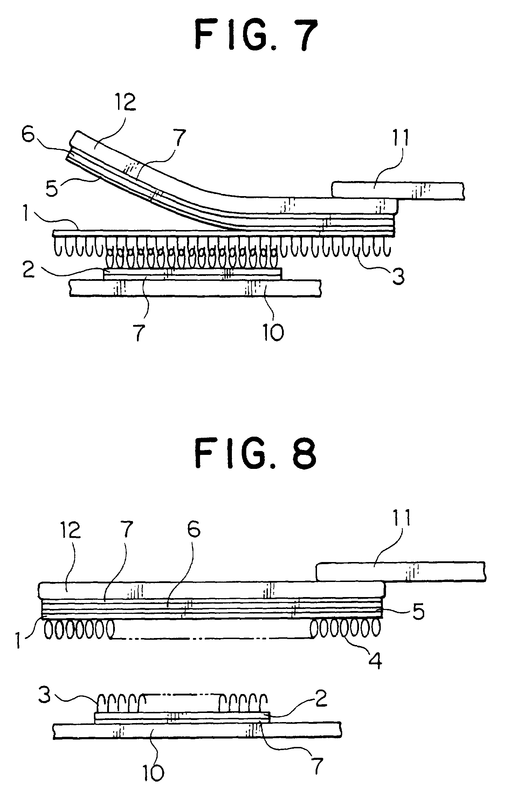

FIGS. 6 and 7 respectively shows another example in which the surface fastener of FIG. 2 is used in a paper diaper A. In this example, the second substrate sheet 2 of each female surface fastener member is attached to each of opposite sides of the front covering portion 10 of the paper diaper A by the adhesive layer 7 with the peel paper 8 peeled off. In the meantime, the first substrate sheet 1 of each male surface fastener member is attached to each of the attachment strips 12 of the hip covering portion 11 of the paper diaper A by the adhesive layer 7 with the peel paper 8 peeled off.

For casting off the paper diaper A after using, one end of the attachment strip 12 is pulled outwardly to separate the adhesive double coated tape 5 from the first substrate sheet 1 so as to stay on the backing sheet 6. After it is folded, the paper diaper A is wrapped by adhering the separated attachment strips 12 to the outer surface of the folded paper diaper A at some positions.

FIG. 8 shows a third embodiment in which a modified surface fastener is used in a paper diaper A. In this modified surface fastener, the male surface fastener member includes a second substrate sheet 2 having on its front surface a multiplicity of hook-shaped engaging elements 3, an adhesive layer 7 on a rear surface of the second substrate sheet 2, and a peel paper 8 adhered to an outer surface of the adhesive layer 7, while the female surface fastener member includes a first substrate sheet 1 having on its front surface a multiplicity of loop-shaped engaging elements 4, an adhesive double coated tape 5 adhered to a rear surface of the substrate sheet 1, a backing sheet 6 adhered to an outer surface of the adhesive double coated tape 5, and another adhesive layer 7 and a peel paper 8 on an outer surface of the backing sheet 6. The male surface fastener member is attached to each of opposite sides of the front covering portion 10 of the paper diaper A by the adhesive layer 7 with the peel paper 8 peeled off the adhesive layer 7. In the meantime, the female surface fastener member is attached to each of the attachment strips 12 of the hip covering portion 11 of the paper diaper A by its adhesive layer 7 with the peel paper 8 peeled off.

FIG. 9 shows a fourth embodiment in which another modified surface fastener is used in a paper diaper A. In this modified surface fastener, the female surface fastener member is identical with that of the previous embodiment, while the male surface fastener member is different from that of the previous embodiment in that the first substrate sheet 1 and the hook-shaped engaging elements 3 are integrally molded by injection molding using thermoplastic resin. For attaching the modified surface fastener to the paper diaper A, likewise in the previous example, the second substrate sheet 2 of each female surface fastener is attached to each of opposite sides of the front covering portion 10 of the paper diaper A by the adhesive layer 7 which is disposed on the rear surface of the second substrate sheet 2.

The molded male surface fastener member has on the rear surface of the first substrate sheet 1 an adhesive double coated tape 5, a backing sheet 6 adhered to an outer surface of the adhesive tape 5, an adhesive layer 7 and a peel paper 8 on an outer surface of the backing sheet 6. In use, with the peel paper 8 peeled off, the male surface fastener member is attached to each attachment strip 12 of the hip covering portion 11 of the paper diaper A by the adhesive layer 7. The used paper diaper A is cast off in the same manner as the previous example.

In manufacturing a surface fastener by weaving or knitting, a surface fastener may be woven or knitted in such a manner that hook-shaped engaging elements 3 and loop-shaped engaging elements 4 are arranged in combination on the front surface of each of the first and second substrate sheets 1, 2. The resulting surface fastener member can be attached to either of a male or female surface fastener on the paper diaper A.

The foregoing surface fasteners and the foregoing paper diapers using the surface fasteners have the following advantageous results:

Partly since the first substrate sheet 1 has on its front surface a multiplicity of first engaging elements 3, 4 and on its rear surface an adhesive layer 7 to an outer surface of which a peel paper 8 is adhered and partly since the second substrate sheet 2 has on its front surface a multiplicity of second engaging elements 3, 4 and on its rear surface an adhesive double coated tape 5 to an outer surface of which a backing sheet 6 is adhered and partly since the adhesive strength of the adhesive double coated tape 5 is smaller than that of the adhesive layer 7, it is possible to separate a garment from the surface fastener with its male and female engaging elements 3, 4 kept in engagement with one another, thus it is possible to handle the surface fastener very simply especially when used in a disposable paper diaper.

Since an adhesive layer 7 and a peel paper 8 are disposed on the backing sheet 6 on a side opposite to the adhesive double coated tape 5, it is possible to handle the surface fastener much more simply especially when used in a disposable paper diaper.

Since the substrate sheet 1, 2 of the female surface fastener member has an adhesive layer 7 while the substrate sheet 2 of the male surface fastener member has an adhesive double coated tape 5, it is possible to separate a garment at the adhesive double coated tape 5 on the male surface fastener member and to make the surface fastener optimal in view of attachment, strength and handling especially when used in a disposable paper diaper.

Since one of the first and second substrate sheets 1, 2 is woven or knitted and has hook-shaped engaging elements 3 and/or loop-shaped engaging elements 4, it is possible to separate a garment from the surface fastener easily with male and female engaging elements 3, 4 kept in engagement with one another and to combine the surface fastener member with any kind of companion surface fastener member.

Since the hook-shaped engaging elements 3 are molded on the substrate sheet 1, 2 by injection molding, it is possible to separate a garment simply with male and female engaging elements 3, 4 kept in engagement with one another.

Partly since the substrate sheet 1, 2 of one surface fastener member is attached to the front covering portion 10 of the paper diaper A by an adhesive layer 7, and partly since the substrate sheet 1, 2 of the other surface fastener member is attached to the attachment strip 12 of the hip covering portion 11 of the paper diaper A by an adhesive double coated tape 5, it is possible to separate a garment with male and female engaging elements 3, 4 kept in engagement with one another, thus preventing the adhesive layer 7 or the adhesive double coated tape 5 from directly touching the infant's skin so that the infant is kept free from skin diseases such as atopic dermatitis.

Since a backing sheet 6 is adhered at one side surface to the adhesive double coated tape 5 and is adhered at the other side surface to the attachment strips 12 of the hip covering portion 11 by an adhesive layer 7, it is possible to handle the paper diaper very simply.

## Claims

1. A surface fastener comprising a first substrate sheet (1) having on its front surface a multiplicity of first engaging elements (3) and on its rear surface a first adhesive layer (7), and a second substrate sheet (2) having on its front surface a multiplicity of second engaging elements (4) engageable with said first engaging elements (3)and on its rear surface a second adhesive layer (7), **characterised in that** the adhesive strength of said first adhesive layer is smaller than that of said second adhesive layer (7).

2. A surface fastener according to claim 1, wherein said first adhesive layer is an adhesive double coated tape (5) and has on its outer surface a backing sheet (6) adhered thereto, and said second adhesive layer (7) has on its outer surface a peel paper adhered thereto.

3. A surface fastener according to claim 2, wherein said backing sheet (6) has on its outer surface another adhesive layer (7) to an outer surface of which another peel paper (8) is adhered.

4. A surface fastener according to claim 1 or 2, wherein said second engaging elements of said second substrate sheet (2) on which the second adhesive layer (7) is disposed are loop-shaped engaging elements (4), and said first engaging elements of said first substrate sheet (1) on which said first adhesive layer is disposed are hook-shaped engaging elements (3).

5. A surface fastener according to claim 1 or 2, wherein at least one of said first and second substrate sheets (1 or 2) is knitted or woven, and said engaging elements of one of said first and second substrate sheets (1 or 2) are hook-shaped engaging elements (3) and/or loop-shaped engaging elements (4).

6. A surface fastener according to claim 4, wherein said hook-shaped engaging elements (3) are molded on said front surface of said first substrate sheet (1) by injection molding.

7. A paper diaper having a front covering portion (10), a hip covering portion (11) having attachment strips (12) on opposite sides thereof, and a plurality of surface fasteners, each surface fastener including a first substrate sheet (1) having on its front surface a multiplicity of first engaging elements (3) and a second substrate sheet (2) having on its front surface a multiplicity of second engaging elements (4) engageable with said first engaging elements (3),
**characterized in that** said second substrate sheet (2) has on its rear surface an adhesive layer (7) which adheres said substrate sheet (2) to said front covering portion (10), and that an adhesive double coated tape (5) is adhered to a rear surface of said first substrate sheet (1) with an adhesive strength which is smaller than that of said adhesive layer (7), said adhesive double coated tape (5) being adhered to a respective attachment strip (12).

8. A paper diaper according to claim 7, wherein a backing sheet (6) is adhered to an outer surface of said adhesive double coated tape (5) and has on a surface opposite to the tape (5) an adhesive layer (7) of which outer surface is adhered the respective attachment strip (12) of said hip covering portion (11).

9. A paper diaper according to claim 7, wherein said second engaging elements (4) of said second substrate sheet (2) on which the first-mentioned adhesive layer (7) is disposed are loop-shaped engaging elements (4), and said first engaging elements of said first substrate sheet (1) on which said adhesive double coated tape (5) is disposed are hook-shaped engaging elements (3).

10. A paper diaper according to claim 7, wherein at least one of said first and second substrate sheets (1 or 2) is knitted or woven, and said engaging elements of one of said first and second substrate sheets (1 or 2) are hook-shaped engaging elements (3) and/or loop-shaped engaging elements (4).

11. A paper diaper according to claim 9, wherein hook-shaped engaging elements (3) are molded on said front surface of said first substrate sheet (1) by injection molding.

## Patentansprüche

1. Flächenhaftverschluss, welcher eine erste Trägermaterialbahn (1), die auf ihrer Vorderseite eine Mehrzahl von ersten Eingreifelementen (3) und auf ihrer Rückseite eine erste Klebstoffschicht (7) aufweist, und eine zweite Trägermaterialbahn (2) beinhaltet, die auf ihrer Vorderseite eine Mehrzahl von zweiten Eingreifelementen (4), die mit den ersten Eingreifelementen (3) in Eingriff gebracht werden können, und auf ihrer Rückseite eine zweite Klebstoffschicht (7) aufweist, **dadurch gekennzeichnet, dass** die Klebfestigkeit der ersten Klebstoffschicht geringer ist als die der zweiten Klebstoffschicht (7).

2. Flächenhaftverschluss nach Anspruch 1, bei welchem die erste Klebstoffschicht ein doppelseitig klebendes Klebeband (5) ist, auf dessen Außenfläche eine Verstärkungsbahn (6) aufgeklebt ist, und auf der Außenfläche der zweiten Klebstoffschicht (7) ein Abziehpapier (8) aufgeklebt ist.

3. Flächenhaftverschluss nach Anspruch 2, bei welchem die Verstärkungsbahn (6) auf ihrer Außenfläche eine weitere Klebstoffschicht (7) aufweist, auf deren Außenfläche ein weiteres Abziehpapier aufgeklebt ist.

4. Flächenhaflverschluss nach Anspruch 1 oder 2, bei welchem die zweiten Eingreifelemente der zweiten Trägermaterialbahn (2), auf welcher die zweite Klebstoffschicht (7) angeordnet ist, schlingenförmige Eingreifelemente (4) sind, und die ersten Eingreifelemente der ersten Trägermaterialbahn (1), auf welcher die erste Klebstoffschicht angeordnet ist, hakenförmige Eingreifelemente (3) sind.

5. Flächenhaftverschluss nach Anspruch 1 oder 2, bei welchem mindestens eine von der ersten und der zweiten Trägermaterialbahn (1 oder 2) gewirkt oder gewebt sind, und die Eingreifelemente von einer von der ersten und der zweiten Trägermaterialbahn (1 oder 2) hakenförmige Eingreifelemente (3) und/oder schlingenförmige Eingreifelemente (4) sind.

6. Flächenhaflverschluss nach Anspruch 4, bei welchem die hakenförmigen Eingreifelemente (3) auf der Vorderseite der ersten Trägermaterialbahn (1) mittels Spritzgießen formgegossen sind.

7. Papierwindel, welche einen vorderen Bedeckungsabschnitt (10), einen Hüftbedeckungsabschnitt (11) mit auf entgegengesetzten Seiten von diesem befindlichen Befestigungsstreifen (12), und eine Mehrzahl von Flächenhaftverschlüssen aufweist, wobei jeder Flächenhaftverschluss eine erste Trägermaterialbahn (1), welche auf ihrer Vorderseite eine Mehrzahl von ersten Eingreifelementen (3) aufweist, und eine zweite Trägermaterialbahn (2) beinhaltet, welche auf ihrer Vorderseite eine Mehrzahl von zweiten Eingreifelementen (4) aufweist, die mit den ersten Eingreifelementen (3) in Eingriff gebracht werden können,
**dadurch gekennzeichnet, dass** die zweite Trägermäterialbahn (2) auf ihrer Rückseite eine Klebstoffschicht (7) aufweist, durch welche die Trägermaterialbahn (2) auf den vorderen Bedeckungsabschnitt (10) aufgeklebt ist, und dass ein doppelseitig klebendes Klebeband (5) mit der Rückseite der ersten Trägermaterialbahn (1) mit einer Klebfestigkeit verklebt ist, welche geringer ist als die der Klebstoffschicht (7), wobei das doppelseitig klebende Klebeband (5) mit einem jeweiligen Befestigungsstreifen verklebt ist.

8. Papierwindel nach Anspruch 7, bei welcher eine Verstärkungsbahn (6) auf eine Außenfläche des doppelseitig klebenden Klebebandes (5) aufgeklebt ist und auf einer dem Band (5) entgegengesetzten Fläche eine Klebstoffschicht (7) aufweist, mit deren Außenfläche der jeweilige Befestigungsstreifen (12) des Hüftbedeckungsabschnitt (11) verklebt ist.

9. Papierwindel nach Anspruch 7, bei welcher die zweiten Eingreifelemente (4) der zweiten Trägermaterialbahn (2), auf welcher die zuerst erwähnte Klebstoffschicht (7) angeordnet ist, schlingenförmige Eingreifelemente (4) sind, und die ersten Eingreifelemente der ersten Trägermaterialbahn (1) auf welcher das doppelseitig klebende Klebeband (5) angeordnet ist, hakenförmige Eingreifelemente (3) sind.

10. Papierwindel nach Anspruch 7, bei welcher mindestens eine von der ersten und der zweiten Trägermaterialbahn (1 oder 2) gewirkt oder gewebt sind, und die Eingreifelemente von einer von der ersten und der zweiten Trägermaterialbahn (1 oder 2) hakenförmige Eingreifelemente (3) und/oder schlingenförmige Eingreifelemente (4) sind.

11. Papierwindel nach Anspruch 9, bei welcher hakenförmige Eingreifelemente (3) auf der Vorderseite der ersten Trägermaterialbahn (1) mittels Spritzgießen formgegossen sind.

## Revendications

1. Fermeture contact comprenant un premier substrat en feuille (1) qui comporte sur sa surface avant une multitude de premiers éléments d'accrochage (3) et sur sa surface arrière une première couche adhésive (7), et un deuxième substrat en feuille (2) qui comporte sur sa surface avant une multitude de deuxièmes éléments d'accrochage (4) pouvant s'accrocher aux dits premiers éléments d'accrochage (3) et sur sa surface arrière une deuxième couche adhésive (7), **caractérisée en ce que** la force d'adhésion de ladite première couche adhésive est inférieure à celle de ladite deuxième couche adhésive (7).

2. Fermeture contact selon la revendication 1, dans laquelle ladite première couche adhésive est un ruban adhésif double face (5) et comporte sur sa surface extérieure une feuille de support (6) qui y adhère, et ladite deuxième couche adhésive (7) comporte sur sa surface extérieure un papier pelable qui y adhère.

3. Fermeture contact selon la revendication 2, dans laquelle ladite feuille de support (6) comporte sur sa surface extérieure une autre couche adhésive (7) à la surface extérieure de laquelle adhère un autre papier pelable (8).

4. Fermeture contact selon la revendication 1 ou 2, dans laquelle lesdits deuxièmes éléments d'accrochage dudit deuxième substrat en feuille (2) sur lequel est placée la deuxième couche adhésive (7) sont des éléments d'accrochage en forme de boucles (4), et lesdits premiers éléments d'accrochage dudit premier substrat en feuille (1) sur lequel est placée ladite première couche adhésive sont des éléments d'accrochage en forme de crochets (3).

5. Fermeture contact selon la revendication 1 ou 2, dans laquelle au moins un desdits premier et deuxième substrats en feuille (1 ou 2) est tricoté ou tissé, et lesdits éléments d'accrochage de l'un desdits premier et deuxième substrats en feuille (1 ou 2) sont des éléments d'accrochage en forme de crochets (3) et/ou des éléments d'accrochage en forme de boucles (4).

6. Fermeture contact selon la revendication 4, dans laquelle lesdits éléments d'accrochage en forme de crochets (3) sont moulés sur ladite surface avant dudit premier substrat en feuille (1) par moulage par injection.

7. Couche-culotte en papier comportant une partie couvrante avant (10), une partie recouvrant les hanches (11) comportant des bandes de fixation (12) sur ses côtés opposés, et une pluralité de fermetures contact, chaque fermeture contact comprenant un premier substrat en feuille (1) qui comporte sur sa surface avant une multitude de premiers éléments d'accrochage (3) et un deuxième substrat en feuille (2) qui comporte sur sa surface avant une multitude de deuxièmes éléments d'accrochage (4) pouvant s'accrocher aux dits premiers éléments d'accrochage (3),
**caractérisée en ce que** ledit deuxième substrat en feuille (2) a, sur sa surface arrière, une couche adhésive (7) qui adhère audit substrat en feuille (2) jusqu'à ladite partie couvrante avant (10), et **en ce qu'**un ruban adhésif double face (5) adhère à une surface arrière dudit premier substrat en feuille (1) avec une force d'adhésion qui est inférieure à celle de ladite couche adhésive (7), ledit ruban adhésif double face (5) étant collé à une bande de fixation respective (12).

8. Couche-culotte en papier selon la revendication 7, dans laquelle une feuille de support (6) est collée à une surface extérieure dudit ruban adhésif double face (5) et comporte sur une surface en vis-à-vis du ruban (5) une couche adhésive (7) dont la surface extérieure est collée à la bande de fixation respective (12) de ladite partie recouvrant les hanches (11).

9. Couche-culotte en papier selon la revendication 7, dans laquelle lesdits deuxièmes éléments d'accrochage (4) dudit deuxième substrat en feuille (2) sur lequel est placée la première couche adhésive mentionnée (7) sont des éléments d'accrochage en forme de boucles (4), et lesdits premiers éléments d'accrochage dudit premier substrat en feuille (1) sur lequel est placé ledit ruban adhésif double face (5) sont des éléments d'accrochage en forme de crochets (3).

10. Couche-culotte en papier selon la revendication 7, dans laquelle au moins un desdits premier et deuxième substrats en feuille (1 ou 2) est tricoté ou tissé, et lesdits éléments d'accrochage de l'un desdits premier et deuxième substrats en feuille (1 ou 2) sont des éléments d'accrochage en forme de crochets (3) et/ou des éléments d'accrochage en forme de boucles (4).

11. Couche-culotte en papier selon la revendication 9, dans laquelle des éléments d'accrochage en forme de crochets (3) sont moulés sur ladite surface avant dudit premier substrat en feuille (1) par moulage par injection.
